# EUROPEAN PATENT APPLICATION

(11) **EP 0 797 999 A2**
(43) Date of publication of application: **01.10.1997**
(21) Application number: 97301995.3
(22) Date of filing: 24.03.1997
(51) Int. Cl.: A61K 38/22, C07K 14/575, C12N 15/16

(54) **Formulations of obesity protein**

(30) Priority: 26.03.1996 US 14177; 05.04.1996 US 14951
(71) Applicant: ELI LILLY AND COMPANY, Indianapolis, Indiana 46285 (US)
(72) Inventor: Beals, John Michael, Indianapolis, Indiana 46278-1853 (US); Edwards, Mary Joanna, Indianapolis, Indiana 46205 (US); Pikal, Michael John, Ashford, Connecticut 06278 (US); Rinella, Joseph Vincent, Jr., Brownsburg, Indiana 46112 (US)
(74) Representative: Hudson, Christopher Mark

(57) **Abstract**

The present invention provides lyophilized compositions of an obesity protein. The compositions demonstrate remarkable stability and ease of manufacture and, significantly, are useful to prepare, stable pharmaceutical formulations useful for treating patients in need thereof. Thus, the invention further provides methods of using such formulations for the treatment of obesity, and disorders associated with obesity such as diabetes, cardiovascular disease and cancer.

## Description

The present invention is directed to lyophilized and soluble formulations of an obesity protein and to methods of using such formulations. More specifically, the invention relates to lyophilized and soluble formulations having increased stability. The present formulations are safe and effective for therapeutic administration to patients in need thereof.

Obesity, and especially upper body obesity, is a common and very serious public health problem in the United States and throughout the world. According to recent statistics, more than 25% of the United States population and 27% of the Canadian population are overweight. Kuczmarski, Amer. J. of Clin. Nutr. 55: 495S - 502S (1992); Reeder et. al., Can. Med. Ass. J., 23: 226-233 (1992). Upper body obesity is the strongest risk factor known for type II diabetes mellitus, and is a strong risk factor for cardiovascular disease and cancer as well. Recent estimates for the medical cost of obesity are $150,000,000,000 world wide. The problem has become serious enough that the surgeon general has begun an initiative to combat the ever increasing adiposity rampant in American society.

Much of this obesity induced pathology can be attributed to the strong association with dyslipidemia, hypertension, and insulin resistance. Many studies have demonstrated that reduction in obesity by diet and exercise reduces these risk factors dramatically. Unfortunately, these treatments are largely unsuccessful with a failure rate reaching 95%. This failure may be due to the fact that the condition is strongly associated with genetically inherited factors that contribute to increased appetite, preference for highly caloric foods, reduced physical activity, and increased lipogenic metabolism. This indicates that people inheriting these genetic traits are prone to becoming obese regardless of their efforts to combat the condition. Therefore, a pharmacological agent that can correct this adiposity handicap and allow the physician to successfully treat obese patients in spite of their genetic inheritance is needed.

The *ob/ob* mouse is a model of obesity and diabetes that is known to carry an autosomal recessive trait linked to a mutation in the sixth chromosome. Recently, Yiying Zhang and co-workers published the positional cloning of the mouse gene linked with this condition. Yiying Zhang et al. Nature 372: 425-32 (1994). This report disclosed the murine and human protein expressed in adipose tissue. Likewise, Murakami et al., in Biochem. and Biophys. Res. comm. 209(3);944-52 (1995) report the cloning and expression of the rat obese gene. The protein, which is encoded by the *ob* gene, has demonstrated an ability to effectively regulate adiposity in mice. Pelleymounter et al., Science 269: 540-543 (1995).

While the native obesity protein is speculated to be the satiety factor associated with obesity, little is known regarding how to formulate the protein for pharmaceutical use. A pharmaceutical formulation of an obesity protein must maintain activity for an appropriate period of time, must be readily manufactureable, and must provide for convenient administration to the patient in need thereof. The present invention provides such formulations.

The present invention provides lyophilized compositions of an obesity protein. The compositions demonstrate remarkable stability and ease of manufacture and, significantly, are useful to prepare, stable pharmaceutical formulations. The formulations are useful for treating patients in need thereof. The invention also provides soluble pharmaceutical formulations of an obesity protein.

The invention further provides methods of using such formulations for the treatment of obesity, and disorders associated with obesity such as diabetes, cardiovascular disease and cancer.

The present invention provides a lyophilized composition, comprising an obesity protein, and a sugar selected from the group consisting of sucrose, trehalose, or a mixture thereof.

The invention further provides a pharmaceutical formulation, comprising the lyophilized composition and water.

The invention further provides a pharmaceutical formulation comprising a sugar selected from the group consisting of sucrose, trehalose, or a mixture thereof, and an obesity protein.

An additional embodiment of the invention is a method of using the formulations of the present invention to treat patients in need thereof.

Yet another embodiment is a therapeutically useful kit, comprising a sterile vial containing a lyophilized composition of the present invention, and a pharmaceutically acceptable diluent for reconstitution thereof. The kit may further comprise a device suitable for injection of the reconstituted preparation by the end user and instructions for reconstitution and optionally for administration.

Figure 1 describes the preparation of the claimed invention.

For purposes of the present invention, as disclosed and claimed herein, the following terms and abbreviations are defined as follows:

Base pair (bp) -- refers to DNA or RNA. The abbreviations A,C,G, and T correspond to the 5'-monophosphate forms of the nucleotides (deoxy)adenine, (deoxy)cytidine, (deoxy)guanine, and (deoxy)thymine, respectively, when they occur in DNA molecules. The abbreviations U,C,G, and T correspond to the 5'-monophosphate forms of the nucleosides uracil, cytidine, guanine, and thymine, respectively when they occur in RNA molecules. In double stranded DNA, base pair may refer to a partnership of A with T or C with G. In a DNA/RNA heteroduplex, base pair may refer to a partnership of T with U or C with G.

Obesity protein or Ob protein -- refers to the protein produced from the obesity gene following transcription and deletions of introns, translation to a protein and processing to the mature protein with secretory signal peptide removed, e.g., from the N-terminal valine-proline to the C-terminal cysteine of the mature protein. The mouse obesity protein and human obesity protein are published in Zhang et al., Nature 372: 425-32 (1994). The rat obesity protein is published in Murakami et al., Biochem. and Biophys. Res. Comm. 209(3): 944-52 (1995). The porcine and bovine obese gene and protein are disclosed in EP O 743 321. Various primate obese genes and proteins are disclosed in U.S. serial number 08/710,483, EP O 764 722. All of which are incorporated by reference. It is understood that the deletion of one or more amino acids in a natural variant or fragment as well as any amino acid additions, including additions to the valine at position 1 (such as a leader sequence), that do not affect the novel and basic characteristics of the invention are included in the definition of Ob protein. A leader sequence is one or more amino acids on the N-terminus to aid in the production or purification of the protein. A preferred leader sequence is Met-R1, wherein R1 is any amino acid except Pro or absent. The term Ob protein also includes various analogs of the Ob protein, some of which have enhanced biological activity or stability. An analog of Ob protein is a native Ob protein having one or more amino acid substitutions, preferably less than five and most preferably less than three substitutions. Ob protein, therefore, includes proteins disclosed by Basinski et al., in WO 96/23515 and WO 96/23517, herein incorporated by reference. More specifically Ob protein includes a protein of the Formula (I): 
(SEQ ID NO: 1)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Xaa Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile
 
65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Trp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys                                                     (I)
 wherein:
Xaa at position 28 is Gln or absent;
said protein having at least one of the following substitutions:
   Gln at position 4 is replaced with Glu;
   Gln at position 7 is replaced with Glu;
   Asn at position 22 is replaced with Gln or Asp;
   Thr at position 27 is replaced with Ala;
   Xaa at position 28 is replaced with Glu;
   Gln at position 34 is replaced with Glu;
   Met at position 54 is replaced with methionine sulfoxide, Leu, Ile, Val, Ala, or Gly;
   Gln at position 56 is replaced with Glu;
   Gln at position 62 is replaced with Glu;
   Gln at position 63 is replaced with Glu;
   Met at position 68 is replaced with methionine sulfoxide, Leu, Ile, Val, Ala, or Gly;
   Asn at position 72 is replaced with Gln, Glu, or Asp;
   Gln at position 75 is replaced with Glu;
   Ser at position 77 is replaced with Ala;
   Asn at position 78 is replaced with Gln or Asp;
   Asn at position 82 is replaced with Gln or Asp;
   His at position 97 is replaced with Gln, Asn, Ala, Gly, Ser, or Pro;
   Trp at position 100 is replaced with Ala, Glu, Asp, Asn, Met, Ile, Phe, Tyr, Ser, Thr, Gly, Gln, Val or Leu;
   Ala at position 101 is replaced with Ser, Asn, Gly, His, Pro, Thr, or Val;
   Ser at position 102 is replaced with Arg;
   Gly at position 103 is replaced with Ala;
   Glu at position 105 is replaced with Gln;
   Thr at position 106 is replaced with Lys or Ser;
   Leu at position 107 is replaced with Pro;
   Asp at position 108 is replaced with Glu;
   Gly at position 111 is replaced with Asp;
   Gly at position 118 is replaced with Leu;
   Gln at position 130 is replaced with Glu;
   Gln at position 134 is replaced with Glu;
   Met at position 136 is replaced with methionine sulfoxide, Leu, Ile, Val, Ala, or Gly;
   Trp at position 138 is replaced with Ala, Glu, Asp, Asn, Met, Ile, Phe, Tyr, Ser, Thr, Gly, Gln, Val or Leu; or
   Gln at position 139 is replaced with Glu;
   or a pharmaceutically acceptable salt thereof.

Plasmid -- an extrachromosomal self-replicating genetic element.

Reading frame -- the nucleotide sequence from which translation occurs "read" in triplets by the translational apparatus of tRNA, ribosomes and associated factors, each triplet corresponding to a particular amino acid. Because each triplet is distinct and of the same length, the coding sequence must be a multiple of three. A base pair insertion or deletion (termed a frameshift mutation) may result in two different proteins being coded for by the same DNA segment. To insure against this, the triplet codons corresponding to the desired polypeptide must be aligned in multiples of three from the initiation codon, i.e. the correct "reading frame" must be maintained. In the creation of fusion proteins containing a chelating peptide, the reading frame of the DNA sequence encoding the structural protein must be maintained in the DNA sequence encoding the chelating peptide.

Recombinant DNA Cloning Vector -- any autonomously replicating agent including, but not limited to, plasmids and phages, comprising a DNA molecule to which one or more additional DNA segments can or have been added.

Recombinant DNA Expression Vector -- any recombinant DNA cloning vector in which a promoter has been incorporated.

Replicon -- A DNA sequence that controls and allows for autonomous replication of a plasmid or other vector.

Transcription -- the process whereby information contained in a nucleotide sequence of DNA is transferred to a complementary RNA sequence.

Translation -- the process whereby the genetic information of messenger RNA is used to specify and direct the synthesis of a polypeptide chain.

Vector -- a replicon used for the transformation of cells in gene manipulation bearing polynucleotide sequences corresponding to appropriate protein molecules which, when combined with appropriate control sequences, confer specific properties on the host cell to be transformed. Plasmids, viruses, and bacteriophage are suitable vectors, since they are replicons in their own right. Artificial vectors are constructed by cutting and joining DNA molecules from different sources using restriction enzymes and ligases. Vectors include Recombinant DNA cloning vectors and Recombinant DNA expression vectors.

Treating -- as used herein, describes the management and care of a patient for the purpose of combating the disease, condition, or disorder and includes the administration of a protein of present invention to prevent the onset of the symptoms or complications, alleviating the symptoms or complications, or eliminating the disease, condition, or disorder. Treating as used herein includes the administration of the protein for cosmetic purposes. A cosmetic purpose seeks to control the weight of a mammal to improve bodily appearance.

Isotonicity agent -- isotonicity agent refers to an agent that is physiologically tolerated and embarks a suitable tonicity to the formulation to prevent the net flow of water across the cell membrane. Compounds, such as glycerin, are commonly used for such purposes at known concentrations. Other possible isotonicity agents include salts, e.g., NaCl, dextrose, and lactose.

Physiologically tolerated buffer -- a physiologically tolerated buffer is known in the art. Physiologically tolerated buffers include TRIS, sodium acetate, sodium phosphate, sodium citrate, arginine or glycine. The selection and concentration of buffer is known in the art.

Pharmaceutically acceptable preservative -- a multi-use parenteral formulation must meet guidelines for preservative effectiveness to be a commercially viable product. Pharmaceutically acceptable preservatives known in the art as being acceptable in parenteral formulations include: phenol, m-cresol, benzyl alcohol, methylparaben, chlorobutanol, p-cresol, phenylmercuric nitrate, thimerosal and various mixtures thereof. Other preservatives may be found, e.g., in WALLHAUSER, K.-H., DEVELOP. BIOL. STANDARD. 24, pp. 9-28 (Basel, S. Krager, 1974). The concentration necessary to achieve preservative effectiveness is dependent upon the preservative used and the conditions of the formulation.

The nucleotide and amino acid abbreviations used herein are accepted by the United States Patent and Trademark Office as set forth in 37 C.F.R. § 1.822 (b) (2) (1993). Unless otherwise indicated the amino acids are in the L configuration.

As noted above, the invention provides a lyophilized and soluble pharmaceutical formulations, comprising an obesity protein, and a sugar selected from the group consisting of sucrose, trehalose, or a mixture thereof.

Preferred proteins of the present invention are those of Formula I, wherein:
Asn at position 22 is replaced with Gln or Asp;
Thr at position 27 is replaced with Ala;
Met at position 54 is replaced with methionine sulfoxide, Leu, or Ala;
Met at position 68 is replaced with methionine sulfoxide, or Leu;
Asn at position 72 is replaced with Gln or Asp;
Asn at position 78 is replaced with Gln or Asp;
Asn at position 82 is replaced with Gln or Asp; or
Met at position 136 is replaced with methionine sulfoxide, Leu, or Ile.

Still yet additional preferred proteins are those of Formula I, wherein:
Asn at position 22 is replaced with Gln or Asp;
Thr at position 27 is replaced with Ala;
Met at position 54 is replaced with Leu, or Ala;
Met at position 68 is replaced with Leu;
Asn at position 72 is replaced with Gln or Asp;
Asn at position 78 is replaced with Gln or Asp;
Asn at position 82 is replaced with Gln or Asp; or
Met at position 136 is replaced with Leu, or Ile.

Preferred species within Formula I include species optionally having a leader sequence and of SEQ ID NO: 2 and SEQ ID NO: 3:

(SEQ ID NO: 2)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Trp Ala Ser Gly Leu Glu Thr Leu Asp Ser Leu Gly Gly

115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys

(SEQ ID NO: 3)
                5                   10                  15
Val Pro Ile Gln Lys Val Gln Asp Asp Thr Lys Thr Leu Ile Lys Thr

            20                  25                  30
Ile Val Thr Arg Ile Asn Asp Ile Ser His Ala Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Trp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys

Most significantly, other preferred proteins of the present invention are specific substitutions to amino acid residues 97 to 111, and/or 138 of the proteins of SEQ ID NO: 1. Accordingly, preferred embodiments are compounds comprising proteins of the Formula I having at least one substitution selected from the group consisting of:
His at position 97 is replaced with Gln, Asn, Ala, Gly, Ser, or Pro;
Trp at position 100 is replaced with Ala, Glu, Asp, Asn, Met, Ile, Phe, Tyr, Ser, Thr, Gly, Gln, Val or Leu;
Ala at position 101 is replaced with Ser, Asn, Gly, His, Pro, Thr, or Val;
Ser at position 102 is replaced with Arg;
Gly at position 103 is replaced with Ala;
Glu at position 105 is replaced with Gln;
Thr at position 106 is replaced with Lys or Ser;
Leu at position 107 is replaced with Pro;
Asp at position 108 is replaced with Glu;
Gly at position 111 is replaced with Asp; or
Trp at position 138 is replaced with Ala, Glu, Asp, Asn, Met, Ile, Phe, Tyr, Ser, Thr, Gly, Gln, Val or Leu;
or a pharmaceutically acceptable salt thereof.

More preferred proteins are those wherein: Trp at position 100 is replaced with Ala, Glu, Asp, Asn, Met, Ile, Phe, Tyr, Ser, Thr, Gly, Gln or Leu.

Most preferred species of the present invention are those proteins having a di-sulfide bond between Cys at position 96 and Cys at position 146. Examples of most preferred species include species of SEQ ID NO: 4-10: 
(SEQ ID NO: 4)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Ala Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro
 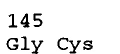 or a pharmaceutically acceptable salt thereof. 
(SEQ ID NO: 5)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Gln Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys
 or a pharmaceutically acceptable salt thereof. 
(SEQ ID NO: 6)
                5                   10                  15
Val Pro Ile Gln Lys Val Gln Asp Asp Thr Lys Thr Leu Ile Lys Thr

            20                  25                  30
Ile Val Thr Arg Ile Asn Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys
 
                100                     105                     110
His Leu Pro Trp Ala Ser Gly Leu Glu Thr Leu Asp Ser Leu Gly Gly

            115                 120                     125
Val Leu Glu Ala Ser Gly Tyr Ser Thr Glu Val Val Ala Leu Ser Arg

    130                     135                 140
Leu Gln Gly Ser Leu Gln Asp Met Leu Gln Gln Leu Asp Leu Ser Pro

145
Gly Cys
 or a pharmaceutically acceptable salt thereof. 
(SEQ ID NO: 7)
                5                   10                  15
Val Pro Ile Gln Lys Val Gln Asp Asp Thr Lys Thr Leu Ile Lys Thr

            20                  25                  30
Ile Val Thr Arg Ile Asn Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Gln Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Gln Gln Leu Asp Leu Ser Pro

145
Gly Cys
 or a pharmaceutically acceptable salt thereof. 
(SEQ ID NO: 8)
5 10 15
Val Pro Ile Gln Lys Val Gln Asp Asp Thr Lys Thr Leu Ile Lys Thr
20 25 30
Ile Val Thr Arg Ile Asn Asp Ile Ser His Ala Gln Ser Val Ser Ser
35 40 45
Lys Gln Lys Val Thr Gly Leu Asp Phe Ile Pro Gly Leu His Pro Ile
 
50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Ala Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys
 or a pharmaceutically acceptable salt thereof. 
(SEQ ID NO: 9)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Ala Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Gln Gln Leu Asp Leu Ser Pro

145
Gly Cys
 or a pharmaceutically acceptable salt thereof. 
(SEQ ID NO: 10)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
Ser Leu Pro Gln Thr Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Gln Gln Leu Asp Leu Ser Pro

145
Gly Cys
 or a pharmaceutically acceptable salt thereof.

The present invention is based on the observation that the presence of a sugar, specifically sucrose and/or trehalose in a formulation of an obesity protein improves the stability of the formulation. That is, in lyophilized formulations, high molecular weight protein (HMWP) formation and dimer formation of the protein is minimized under the conditions described herein. In soluble formulations, the degree of aggregation is significantly diminished.

Stability can be either chemical or physical in nature. Chemical stability in protein pharmaceuticals is a function of the propensity of amino acid side chains and the protein backbone to undergo a number of degradation processes that include, but are not limited to, deamidation (e.g., Asn, Gln), high molecular weight polymer formation (e.g., Cys, N-terminal amino groups), oxidation (e.g., Met, Cys), and primary sequence clips (e.g., Asp-Pro). The extent of chemical degradation is directly related to the conditions the protein is exposed to and the duration of the exposure. Chemical stability can be accelerated by changes in the protein environment, e.g., temperature, moisture, and pH. The ability to control chemical degradation with time provides a competitive advantage by improving the quality of the pharmaceutical product received by the patient, enabling larger purification and processing scales, and allowing the patient increased flexibility in the use of the product.

Physical stability in protein pharmaceuticals is associated with gelation, aggregation or precipitation, and/or flocculation that can occur in soluble or suspension products. The extent of the physical degradation is a function of the conditions the protein/product is exposed to and the duration of the exposure. Physical stability can be accelerated by changes in the protein environment, e.g., temperature, moisture, ionic strength, and pH. The ability to control physical degradation provides a competitive advantage by improving the quality of the pharmaceutical product received by the patient, enabling larger purification and processing scales, and allowing the patient increased flexibility in the use of the product.

Remarkably, obesity protein is exceptionally stable in the presence of sucrose, trehalose or mixtures thereof, but notably less stable as a neat or in the presence of mannitol and/or tween known excipients in protein formulations. The stability of the lyophilized formulations described herein is demonstrated in Table 1. The stability of the soluble formulations described herein is demonstrated in Table 2. In Table 2, stability is assessed by the relative absence of aggregated protein. The degree of aggregation is inferred by measuring the turbidity of the formulation. The greater the turbidity of the formulation, the greater the extent of aggregation. Turbidity is commonly determined by nephelometry and measured in Nephalometric Turbidity Units (NTU). Solubility of the protein may also be determined directly by measuring soluble protein concentration by other well-known methodology including spectrophotometry, dye-binding protein assay, or Dynamic Light Scattering (DLS).

The stability of the present formulations is demonstrated in Table 1:

**Table 1:**

| High molecular weight protein, dimer, and main peak results of a protein of SEQ ID NO: 4. Each lyophilized formulation contained 6 mg of excipient, 1.5 mg of protein, and approximately 0.02 g of Na₂HPO₄ at pH 7.8. Tween was added at 0.05%. | | | | | | | | | |
|---|---|---|---|---|---|---|---|---|---|
| Excipient | % Δ monomer/wk | | | % Δ dimer/wk | | | % Δ HMWP/wk | | |
| | 4°C | 40°C | 50°C | 4°C | 40°C | 50°C | 4°C | 40°C | 50°C |
| Neat | 0.0 | -0.5 | -1.0 | 0.0 | 0.4 | 1.0 | 0.0 | 0.0 | 0.1 |
| | (nd) | (±0.0) | (±0.1) | (nd) | (±0.0) | (±0.1) | (nd) | (±0.0) | (±0.0) |
| Mannitol/ Tween | 0.0 | -0.3 | -0.8 | 0.0 | 0.4 | 0.8 | 0.0 | 0.0 | 0.1 |
| | (nd) | (±0.0) | (±0.0) | (nd) | (±0.1) | (±0.0) | (nd) | (±0.0) | (±0.0) |
| Mannitol | 0.0 | -0.3 | -0.7 | 0.0 | 0.3 | 0.7 | 0.0 | 0.0 | 0.1 |
| | (nd) | (±0.0) | (±0.1) | (nd) | (±0.1) | (±0.0) | (nd) | (±0.0) | (±0.0) |
| Trehalose | 0.0 | 0.0 | -0.1 | 0.0 | 0.0 | 0.1 | 0.0 | 0.0 | 0.0 |
| | (nd) | (±0.0) | (±0.0) | (nd) | (±0.0) | (±0.0) | (nd) | (±0.0) | (±0.0) |
| Sucrose | 0.0 | 0.0 | 0.0 | 0.0 | 0.0 | 0.0 | 0.0 | 0.0 | 0.0 |
| | (nd) | (±0.1) | (±0.1) | (nd) | (±0.0) | (±0.0) | (nd) | (±0.0) | (±0.0) |
| (nd) represents not determined. | | | | | | | | | |

The data in Table 1 demonstrate the remarkable stability of the obesity protein in the presence of trehalose and preferably sucrose.

**Table 2:**

| Effect of sucrose on the turbidity of a soluble formulation comprising the protein of SEQ ID NO:4 at various temperatures. Besides sucrose, each formulation contained 10 mg/mL protein and 5 mM arginine buffer (pH 8.5). Turbidity of the formulations was measured by nephelometry using a Hach Ratio Turbidimeter (Model 18900, or 2100N for values above 200) calibrated with Hach Formazin Turbidity Standard, 4000 NTU (Hach 2461-11, or equivalent). Values are Nephelometric Turbidity Units (NTU) which are sample reading minus diluent blank reading. | | | | |
|---|---|---|---|---|
| Excipient | Turbidity (NTU) | | | |
| 15 % Sucrose | Day 1 | Day 4 | Day 7 | Day 14 |
| 5° C | 1.0 | 1.9 | 2.4 | 3.3 |
| 25° C | 4.4 | 18.8 | 29.2 | 49.8 |
| 37° C | 46.8 | 56.7 | 53.3 | 54.7 |

| 9 % Sucrose | Day 1 | Day 4 | Day 8 | Day 14 |
|---|---|---|---|---|
| 5° C | 2.6 | 3.2 | 4.3 | 5.3 |
| 25° C | 8.0 | 27.5 | 53.6 | 73.9 |
| 37° C | 54.5 | 59.6 | 65.9 | 65.0 |
| Control | | | | |
| 5° C | 3.6 | 5.5 | 7.3 | 9.0 |
| 25° C | 11.7 | 41.5 | 72.1 | 89.6 |
| 37° C | 60.3 | 64 | 72 | 71.3 |
| 5 % Mannitol | | | | |
| 5° C | 5.19 | 9.54 | 14.4 | 18.2 |
| 25° C | 28.5 | 67.9 | 93 | 102 |
| 37° C | 72.1 | 74 | 83.8 | 84 |

The claimed compositions are prepared in a using conventional dissolution, mixing and lyophilization techniques. Generally, the lyophilized composition is prepared by a process comprising combining water, an obesity protein, and a sugar selected from the group consisting of consisting of sucrose, trehalose, or a mixture thereof; and lyophilizing the resulting solution to yield a product incorporating less than 3%, and preferably less than 1%, water by weight. For example, water, sugar, and protein are combined at a pH from about 7.0 to 8.5, preferable pH 7.0 to 7.8 and stirred until the protein and sugar are in solution. When the sugar is trehalose, the formulation may be prepared under acidic conditions, i.e., in a pH range from pH 4.0 to pH 7.0. The solution is then lyophilized producing the claimed lyophilized composition.

The weight ratio of obesity protein to sugar in the claimed composition is 1:0.5 to 1:20, preferably 1:1 to 1:15, more preferably 1:1 to 1:10 and most preferably 1:1 to 1:6. In a preferred embodiment the protein-sugar solution that is contains a physiologically acceptable buffer. Preferred buffer is phosphate buffer; most preferably sodium phosphate. The order in which the protein, buffer, and sugar are added is not critical to the present invention. Furthermore, one skilled in the art would recognize that the solution that is lyophilized may contain other components in amounts not detracting from the stability of the present compositions.

The resulting composition is reconstituted with water prior to administration. Preferably, the composition is reconstituted in a diluent comprising water, a pharmaceutically acceptable buffer and an isotonicity agent at a pH from about 7.0 to 8.5, preferably pH 7.8. The compositions are formulated and administered individually or in combination with other therapeutic agents.

The present invention also includes a pharmaceutical formulation, comprising the lyophilized composition and water. In a preferred embodiment the pharmaceutical formulations of the present invention additionally comprise a pharmaceutically acceptable buffer, an isotonicity agent and, optionally, a pharmaceutically acceptable preservative. A pharmaceutically acceptable preservative is at a concentration necessary to maintain preservative effectiveness. The relative amounts of preservative necessary to maintain preservative effectiveness varies with the preservative used. Generally, the amount necessary can be found in WALLHAUSER, K.-H., DEVELOP. BIOL. STANDARD. 24, pp. 9-28 (Basel, S. Krager, 1974), herein incorporated by reference.

An isotonicity agent, preferably glycerin, is generally added to the formulation. The concentration of the isotonicity agent is in the range known in the art for parenteral formulations, preferably about 16 mg/mL glycerin. The pH of the formulation may also be buffered with a physiologically tolerated buffer. Acceptable physiologically tolerated buffers include TRIS, sodium acetate, sodium phosphate, or sodium citrate. The selection and concentration of buffer is known in the art and may be present in the diluent and/or in lyophilized solid prior to reconstitution.

Other additives, such as pharmaceutically acceptable excipients like Tween 20 (polyoxyethylene (20) sorbitan monolaurate), Tween 40 (polyoxyethylene (20) sorbitan monopalmitate), Tween 80 (polyoxyethylene (20) sorbitan monooleate), Pluronic F68 (polyoxyethylene polyoxypropylene block copolymers), BRIJ 35 (polyoxyethylene (23) lauryl ether), and PEG (polyethylene glycol) may optionally be added to the formulation to reduce aggregation.

When reconstituted, the formulation preferably comprises an obesity protein about 0.1 to 10 mg/mL; sucrose, trehalose or a mixture thereof at a concentration of about 0.6 to 60 mg/mL; and preferably, sodium phosphate at a concentration of 14 mM and glycerin at a concentration of 16 mg/mL. The most preferred formulation is 10 mg/mL protein, 60 mg/mL sucrose, 14 mM phosphate, 16 mg/mL glycerin. The preferred pH of the formulation is pH 7.0 to 8.5 preferably 7.8.

The preparation of the formulations of the present invention is further illustrated in Figure 1.

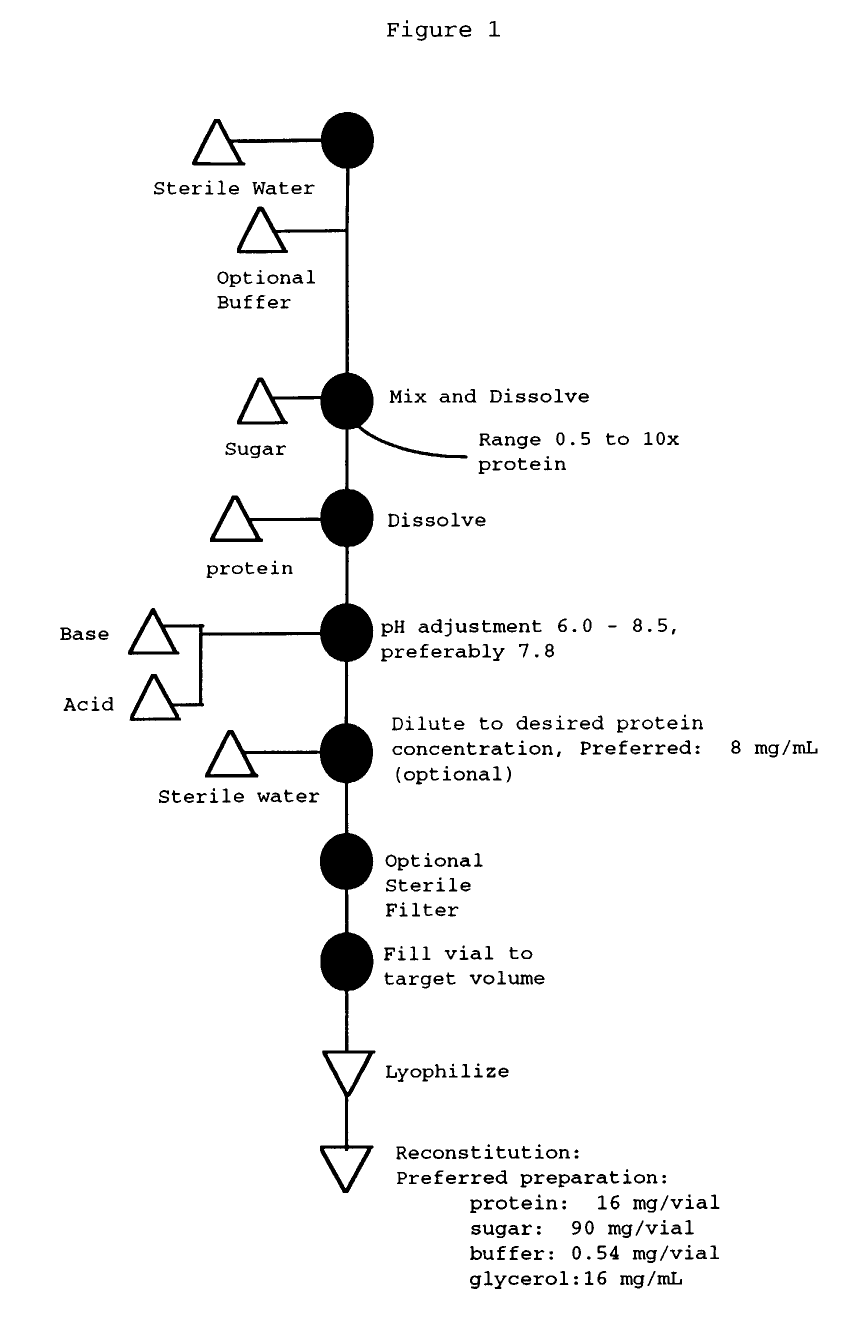
Figure 1


Sterile Water

Optional
Buffer

Mix and Dissolve

Sugar

Range 0.5 to 10x
protein

Dissolve

protein

Base

pH adjustment 6.0 - 8.5,
preferably 7.8

Acid

Dilute to desired protein
concentration, Preferred: 8 mg/mL
(optional)

Sterile water

Optional
Sterile
Filter

Fill vial to
target volume

Lyophilize

Reconstitution:
Preferred preparation:
protein: 16 mg/vial
sugar: 90 mg/vial
buffer: 0.54 mg/vial
glycerol:16 mg/mL

The obesity protein preparations of the present invention are complete in the sense that the end-user need reconstitute the composition solely in sterile water to generate an administerable formulation. For this purpose and in accordance with another aspect of this invention, there is provided a therapeutically useful kit, comprising a sterile vial containing a lyophilized composition of the present invention, and a pharmaceutically acceptable diluent for reconstitution thereof. The kit may further comprise a device suitable for injection of the reconstituted preparation by the end user and instructions for reconstitution and optionally for administration. A pharmaceutically acceptable diluent is generally known to those of ordinary skill in the art. A preferred diluent comprises sterile water, an isotonicity agent, a physiologically tolerated buffer, and optionally a pharmaceutically acceptable preservative. A suitable injection device is a hypodermic needle, for example a 25 gauge needle and a syringe capable of receiving a solution volume of about 0.5 to 5 mL or larger volumes if multiple dose formulations are desired.

In use, the end-user draws diluent into the injection device and transfers the diluent into the vial containing the lyophilized compositions to cause mixing and reconstitution of the solid. Generally, the compositions are mixed until visibly dissolved. After mixing, the end-user injects the formulation in the manner and amount prescribed by the physician.

Parenteral daily doses of the compound are in the range from about 1 ng to about 10 mg per kg of body weight, although lower or higher dosages may be administered. The required dosage will be determined by the physician and will depend on the severity of the condition of the patient and upon such criteria as the patient's height, weight, sex, age, and medical history.

The proteins used in the present invention can be prepared by any of a variety of recognized peptide synthesis techniques including classical (solution) methods, solid phase methods, semi synthetic methods, and more recent recombinant DNA methods. Recombinant methods are preferred if a high yield is desired. The basic steps in the recombinant production of protein include:
a) construction of a synthetic or semi-synthetic (or isolation from natural sources) DNA encoding the obesity protein,
b) integrating the coding sequence into an expression vector in a manner suitable for the expression of the protein either alone or as a fusion protein,
c) transforming an appropriate eukaryotic or prokaryotic host cell with the expression vector, and
d) recovering and purifying the recombinantly produced protein.
Synthetic genes, the in vitro or in vivo transcription and translation of which will result in the production of the protein may be constructed by techniques well known in the art. Owing to the natural degeneracy of the genetic code, the skilled artisan will recognize that a sizable yet definite number of DNA sequences may be constructed which encode the desired proteins. In the preferred practice of the invention, synthesis is achieved by recombinant DNA technology.

Methodology of synthetic gene construction is well known in the art. For example, see Brown, et al. (1979) Methods in Enzymology, Academic Press, N.Y., Vol. 68, pgs. 109-151. The DNA sequence corresponding to the synthetic claimed protein gene may be generated using conventional DNA synthesizing apparatus such as the Applied Biosystems Model 380A or 380B DNA synthesizers (commercially available from Applied Biosystems, Inc., 850 Lincoln Center Drive, Foster City, CA 94404). It may be desirable in some applications to modify the coding sequence of the obesity protein so as to incorporate a convenient protease sensitive cleavage site, e.g., between the signal peptide and the structural protein facilitating the controlled excision of the signal peptide from the fusion protein construct.

The gene encoding the obesity protein may also be created by using polymerase chain reaction (PCR). The template can be a cDNA library (commercially available from CLONETECH or STRATAGENE) or mRNA isolated from the desired arrival adipose tissue. Such methodologies are well known in the art Maniatis, et al. Molecular Cloning: A Laboratory manual, Cold Spring Harbor Press, Cold Spring Harbor Laboratory, Cold Spring Harbor, New York (1989).

The constructed or isolated DNA sequences are useful for expressing the obesity protein either by direct expression or as fusion protein. When the sequences are used in a fusion gene, the resulting product will require enzymatic or chemical cleavage. A variety of peptidases which cleave a polypeptide at specific sites or digest the peptides from the amino or carboxy termini (e.g. diaminopeptidase) of the peptide chain are known. Furthermore, particular chemicals (e.g. cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. See U.S. Patent No. 5,126,249; Carter P., Site Specific Proteolysis of Fusion Proteins, Ch. 13 in Protein Purification: From Molecular Mechanisms to Large Scale Processes, American Chemical Soc., Washington, D.C. (1990).

Construction of suitable vectors containing the desired coding and control sequences employ standard ligation techniques. Isolated plasmids or DNA fragments are cleaved, tailored, and religated in the form desired to form the plasmids required.

To effect the translation of the desired protein, one inserts the engineered synthetic DNA sequence in any of a plethora of appropriate recombinant DNA expression vectors through the use of appropriate restriction endonucleases. A synthetic coding sequence may be designed to possess restriction endonuclease cleavage sites at either end of the transcript to facilitate isolation from and integration into these expression and amplification and expression plasmids. The isolated cDNA coding sequence may be readily modified by the use of synthetic linkers to facilitate the incorporation of this sequence into the desired cloning vectors by techniques well known in the art. The particular endonucleases employed will be dictated by the restriction endonuclease cleavage pattern of the parent expression vector to be employed. The restriction sites are chosen so as to properly orient the coding sequence with control sequences to achieve proper in-frame reading and expression of the protein.

In general, plasmid vectors containing promoters and control sequences which are derived from species compatible with the host cell are used with these hosts. The vector ordinarily carries a replication origin as well as marker sequences which are capable of providing phenotypic selection in transformed cells. For example, E. coli is typically transformed using pBR322, a plasmid derived from an E. coli species (Bolivar, et al., Gene 2: 95 (1977)). Plasmid pBR322 contains genes for ampicillin and tetracycline resistance and thus provides easy means for identifying transformed cells. The pBR322 plasmid, or other microbial plasmid must also contain or be modified to contain promoters and other control elements commonly used in recombinant DNA technology.

The desired coding sequence is inserted into an expression vector in the proper orientation to be transcribed from a promoter and ribosome binding site, both of which should be functional in the host cell in which the protein is to be expressed. An example of such an expression vector is a plasmid described in Belagaje et al., U.S. patent No. 5,304,493, the teachings of which are herein incorporated by reference. The gene encoding A-C-B proinsulin described in U.S. patent No. 5,304,493 can be removed from the plasmid pRB182 with restriction enzymes NdeI and BamHI. The isolated DNA sequences can be inserted into the plasmid backbone on a NdeI/BamHI restriction fragment cassette.

In general, procaryotes are used for cloning of DNA sequences in constructing the vectors useful in the invention. For example, E. coli K12 strain 294 (ATCC No. 31446) is particularly useful. Other microbial strains which may be used include E. coli B and E. coli X1776 (ATCC No. 31537). These examples are illustrative rather than limiting.

Procaryotes also are used for expression. The aforementioned strains, as well as E. coli W3110 (prototrophic, ATCC No. 27325), bacilli such as Bacillus subtilis, and other enterobacteriaceae such as Salmonella typhimurium or Serratia marcescans, and various pseudomonas species may be used. Promoters suitable for use with prokaryotic hosts include the β-lactamase (vector pGX2907 [ATCC 39344] contains the replicon and β-lactamase gene) and lactose promoter systems (Chang et al., Nature, 275:615 (1978); and Goeddel et al., Nature 281:544 (1979)), alkaline phosphatase, the tryptophan (trp) promoter system (vector pATH1 [ATCC 37695] is designed to facilitate expression of an open reading frame as a trpE fusion protein under control of the trp promoter) and hybrid promoters such as the tac promoter (isolatable from plasmid pDR540 ATCC-37282). However, other functional bacterial promoters, whose nucleotide sequences are generally known, enable one of skill in the art to ligate them to DNA encoding the protein using linkers or adaptors to supply any required restriction sites. Promoters for use in bacterial systems also will contain a Shine-Dalgarno sequence operably linked to the DNA encoding protein.

The DNA molecules may also be recombinantly produced in eukaryotic expression systems. Preferred promoters controlling transcription in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. β-actin promoter. The early and late promoters of the SV40 virus are conveniently obtained as an SV40 restriction fragment which also contains the SV40 viral origin of replication. Fiers, et al., Nature, 273:113 (1978). The entire SV40 genome may be obtained from plasmid pBRSV, ATCC 45019. The immediate early promoter of the human cytomegalovirus may be obtained from plasmid pCMBb (ATCC 77177). Of course, promoters from the host cell or related species also are useful herein.

Transcription of the DNA by higher eucaryotes is increased by inserting an enhancer sequence into the vector. Enhancers are cis-acting elements of DNA, usually about 10-300 bp, that act on a promoter to increase its transcription. Enhancers are relatively oriented and positioned independently and have been found 5' (Laimins, L. et al., PNAS 78:993 (1981)) and 3' (Lusky, M. L., et al., Mol. Cell Bio. 3:1108 (1983)) to the transcription unit, within an intron (Banerji, J. L. et al., Cell 33:729 (1983)) as well as within the coding sequence itself (Osborne, T. F., et al., Mol. Cell Bio. 4:1293 (1984)). Many enhancer sequences are now known from mammalian genes (globin, RSV, SV40, EMC, elastase, albumin, alpha-fetoprotein and insulin). Typically, however, one will use an enhancer from a eukaryotic cell virus. Examples include the SV40 late enhancer, the cytomegalovirus early promoter enhancer, the polyoma enhancer on the late side of the replication origin, and adenovirus enhancers.

Expression vectors used in eukaryotic host cells (yeast, fungi, insect, plant, animal, human or nucleated cells from other multicellular organisms) will also contain sequences necessary for the termination of transcription which may affect mRNA expression. These regions are transcribed as polyadenylated segments in the untranslated portion of the mRNA encoding protein. The 3' untranslated regions also include transcription termination sites.

Expression vectors may contain a selection gene, also termed a selectable marker. Examples of suitable selectable markers for mammalian cells are dihydrofolate reductase (DHFR, which may be derived from the BglII/HindIII restriction fragment of pJOD-10 [ATCC 68815]), thymidine kinase (herpes simplex virus thymidine kinase is contained on the BamHI fragment of vP-5 clone [ATCC 2028]) or neomycin (G418) resistance genes (obtainable from pNN414 yeast artificial chromosome vector [ATCC 37682]). When such selectable markers are successfully transferred into a mammalian host cell, the transfected mammalian host cell can survive if placed under selective pressure. There are two widely used distinct categories of selective regimes. The first category is based on a cell's metabolism and the use of a mutant cell line which lacks the ability to grow without a supplemented media. Two examples are: CHO DHFR⁻ cells (ATCC CRL-9096) and mouse LTK⁻ cells (L-M(TK-) ATCC CCL-2.3). These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in nonsupplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, Southern P. and Berg, P., J. Molec. Appl. Genet. 1: 327 (1982), mycophenolic acid, Mulligan, R. C. and Berg, P. Science 209:1422 (1980), or hygromycin, Sugden, B. et al., Mol Cell. Biol. 5:410-413 (1985). The three examples given above employ bacterial genes under eukaryotic control to convey resistance to the appropriate drug G418 or neomycin (geneticin), xgpt (mycophenolic acid) or hygromycin, respectively.

A preferred vector for eucaryotic expression is pRc/CMV. pRc/CMV is commercially available from Invitrogen Corporation, 3985 Sorrento Valley Blvd., San Diego, CA 92121. To confirm correct sequences in plasmids constructed, the ligation mixtures are used to transform E. coli K12 strain DH10B (ATCC 31446) and successful transformants selected by antibiotic resistance where appropriate. Plasmids from the transformants are prepared, analyzed by restriction and/or sequence by the method of Messing, et al., Nucleic Acids Res. 9:309 (1981).

Host cells may be transformed with the expression vectors of this invention and cultured in conventional nutrient media modified as is appropriate for inducing promoters, selecting transformants or amplifying genes. The culture conditions, such as temperature, pH and the like, are those previously used with the host cell selected for expression, and will be apparent to the ordinarily skilled artisan. The techniques of transforming cells with the aforementioned vectors are well known in the art and may be found in such general references as Maniatis, et al., Molecular Cloning: A Laboratory Manual, Cold Spring Harbor Press, Cold Spring Harbor Laboratory, Cold Spring Harbor, New York (1989), or Current Protocols in Molecular Biology (1989) and supplements.

Preferred suitable host cells for expressing the vectors encoding the claimed proteins in higher eucaryotes include: African green monkey kidney line cell line transformed by SV40 (COS-7, ATCC CRL-1651); transformed human primary embryonal kidney cell line 293, (Graham, F. L. et al., J. Gen Virol. 36:59-72 (1977), Virology 77:319-329, Virology 86:10-21); baby hamster kidney cells (BHK-21(C-13), ATCC CCL-10, Virology 16:147 (1962)); Chinese hamster ovary cells CHO-DHFR⁻ (ATCC CRL-9096), mouse Sertoli cells (TM4, ATCC CRL-1715, Biol. Reprod. 23:243-250 (1980)); African green monkey kidney cells (VERO 76, ATCC CRL-1587); human cervical epitheloid carcinoma cells (HeLa, ATCC CCL-2); canine kidney cells (MDCK, ATCC CCL-34); buffalo rat liver cells (BRL 3A, ATCC CRL-1442); human diploid lung cells (WI-38, ATCC CCL-75); human hepatocellular carcinoma cells (Hep G2, ATCC HB-8065);and mouse mammary tumor cells (MMT 060562, ATCC CCL51).

In addition to prokaryotes, unicellular eukaryotes such as yeast cultures may also be used. Saccharomyces cerevisiae, or common baker's yeast is the most commonly used eukaryotic microorganism, although a number of other strains are commonly available. For expression in Saccharomyces, the plasmid YRp7, for example, (ATCC-40053, Stinchcomb, et al., Nature 282:39 (1979); Kingsman et al., Gene 7:141 (1979); Tschemper et al., Gene 10:157 (1980)) is commonly used. This plasmid already contains the trp gene which provides a selection marker for a mutant strain of yeast lacking the ability to grow in tryptophan, for example ATCC no. 44076 or PEP4-1 (Jones, Genetics 85:12 (1977)).

Suitable promoting sequences for use with yeast hosts include the promoters for 3-phosphoglycerate kinase (found on plasmid pAP12BD ATCC 53231 and described in U.S. Patent No. 4,935,350, June 19, 1990) or other glycolytic enzymes such as enolase (found on plasmid pACl ATCC 39532), glyceraldehyde-3-phosphate dehydrogenase (derived from plasmid pHcGAPC1 ATCC 57090, 57091), zymomonas mobilis (United States Patent No. 5,000,000 issued March 19, 1991), hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which contain inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein (contained on plasmid vector pCL28XhoLHBPV ATCC 39475, United States Patent No. 4,840,896), glyceraldehyde 3-phosphate dehydrogenase, and enzymes responsible for maltose and galactose (GAL1 found on plasmid pRY121 ATCC 37658) utilization. Suitable vectors and promoters for use in yeast expression are further described in R. Hitzeman et al., European Patent Publication No. 73,657A. Yeast enhancers such as the UAS Gal from Saccharomyces cerevisiae (found in conjunction with the CYC1 promoter on plasmid YEpsec--hI1beta ATCC 67024), also are advantageously used with yeast promoters.

### Preparation 1

The plasmid containing the DNA sequence encoding the desired protein, is digested with PmlI and Bsu36I. The recognition sequences for these enzymes lie within the coding region for the protein at nucleotide positions 275 and 360 respectively. The cloning vector does not contain these recognition sequences. Consequently, only two fragments are seen following restriction enzyme digestion with PmlI and Bsu36I, one corresponding to the vector fragment, the other corresponding to the -85 base pair fragment liberated from within the protein coding sequence. This sequence can be replaced by any DNA sequence encoding the amino acid substitutions between positions 91 and 116 of the present invention. These DNA sequences are synthesized chemically as two oligonucleotides with complementary bases and ends that are compatible with the ends generated by digestion with PmlI and Bsu36I. The chemically synthesized oligonucleotides are mixed in equimolar amounts (1-10 picomoles/microliter), heated to 95 degrees and allow to anneal by slowly decreasing the temperature to 20-25 degrees. The annealed oligonucleotides are used in a standard ligation reaction. Ligation products are transformed and analyzed as described in Example 1. Other substitutions are preferably carried out in a similar manner using appropriate restriction cites.

### Preparation 2

A DNA sequence encoding SEQ ID NO: 4 with a Met Arg leader sequence was obtained using the plasmid and procedures described in preparation 1. The plasmid was digested with Pm1I and Bsu36I. A synthetic DNA fragment of the sequence 5"-SEQ ID NO:11: 
(SEQ ID NO: 11)
GTGCTGGCCTTCTCTAAAAGTTGCCACTTGCCAGCTGCCAGTGGCCTGGAGACATTGGACA
GTCTGGGGGGAGTCCTGGAAGCC
 annealed with the sequence 5'-SEQ ID NO:12: 
(SEQ ID NO: 12)
TGAGGCTTCCAGGACTCCCCCAGACTGTCCAATGTCTCCAGGCCACTGGCAGCTGGCAAG
TGGCAACTTTTAGAGAAGGCCAGCAC
 was inserted between the PmlI and the Bsu36I sites. Following ligation, transformation and plasmid isolation, the sequence of the synthetic fragment was verified by DNA sequence analysis.

The techniques of transforming cells with the aforementioned vectors are well known in the art and may be found in such general references as Maniatis, et al. (1988) Molecular Cloning: A Laboratory Manual, Cold Spring Harbor Press, Cold Spring Harbor Laboratory, Cold Spring Harbor, New York or Current Protocols in Molecular Biology (1989) and supplements. The techniques involved in the transformation of E. coli cells used in the preferred practice of the invention as exemplified herein are well known in the art. The precise conditions under which the transformed E. coli cells are cultured is dependent on the nature of the E. coli host cell line and the expression or cloning vectors employed. For example, vectors which incorporate thermoinducible promoter-operator regions, such as the c1857 thermoinducible lambda-phage promoter-operator region, require a temperature shift from about 30 to about 40 degrees C. in the culture conditions so as to induce protein synthesis.

In the preferred embodiment of the invention E. coli K12 RV308 cells are employed as host cells but numerous other cell lines are available such as, but not limited to, E. coli K12 L201, L687, L693, L507, L640, L641, L695, L814 (E. coli B). The transformed host cells are then plated on appropriate media under the selective pressure of the antibiotic corresponding to the resistance gene present on the expression plasmid. The cultures are then incubated for a time and temperature appropriate to the host cell line employed.

Proteins that are expressed in high-level bacterial expression systems characteristically aggregate in granules or inclusion bodies which contain high levels of the overexpressed protein. Kreuger et al., in Protein Folding, Gierasch and King, eds., pgs 136-142 (1990), American Association for the Advancement of Science Publication No. 89-18S, Washington, D.C. Such protein aggregates must be dissolved to provide further purification and isolation of the desired protein product. Id. A variety of techniques using strongly denaturing solutions such as guanidinium-HCl and/or weakly denaturing solutions such as urea are used to solubilize the proteins. Gradual removal of the denaturing agents (often by dialysis) in a solution allows the denatured protein to assume its native conformation. The particular conditions for denaturation and folding are determined by the particular protein expression system and/or the protein in question.

### Preparation 3

The protein of SEQ ID NO: 4 with a Met Arg leader sequence was expressed in E.coli granules were isolated in 8M urea and 5mM cysteine. The protein was purified by anion exchange chromatography in 8M urea, and folded by dilution into 8M urea (containing 5 mM cysteine) and exhaustive dialysis against PBS. Following final purification of the proteins by size exclusion chromatography the proteins were concentrated to 3-3.5 mg/mL in PBS.

### Preparation 4

A DNA sequence encoding the protein of SEQ ID NO: 2 was assembled from chemically synthesized single stranded oligonucleotides to generate a double stranded DNA sequence. The oligonucleotides used to assemble this DNA sequence are as follows: 
(SEQ ID NO: 13)
TATGAGGGTACCTATCCAAAAAGTACAAGATGACACCAAAACACTGATAAAGACAATAGTC
ACAAG
(SEQ ID NO: 14)
GATAGATGATATCTCACACACACAGTCAGTCTCATCTAAACAGAAAGTCACAGGCTTGGAC
TTCATACCTGG
(SEQ ID NO: 15)
GCTGCACCCCATACTGACATTGTCTAAAATGGACCAGACACTGGCAGTCTATCAACAGATC
TTAACAAGTATGCCTT
(SEQ ID NO: 16)
CTAGAAGGCATACTTGTTAAGATCTGTTGATAGACTGC
(SEQ ID NO: 17)
CAGTGTCTGGTCCATTTTAGACAATGTCAGTATGGGGTGCAGCCCAGGTATGAAGTCCAAGC
(SEQ ID NO: 18)
CTGTGACTTTCTGTTTAGATGAGACTGACTGTGTGTGTGAGATATCATCTATCCTTGTGAC
TATTGTCTTTATCAGTGTTTTG
(SEQ ID NO: 19)
GTGTCATCTTGTACTTTTTGGATAGGTACCCTCA
(SEQ ID NO: 20)
CTAGAAACGTGATACAAATATCTAACGACCTGGAGAACCTGCGGGATCTGCTGCACGTGCT
GGCCTTCTCTAAAAGTTGCCACTTGCCATGG
(SEQ ID NO: 21)
GCCAGTGGCCTGGAGACATTGGACAGTCTGGGGGGAGTCCTGGAAGCCTCAGGCTATTCTA
CAGAGGTGGTGGC
(SEQ ID NO: 22)
CCTGAGCAGGCTGCAGGGGTCTCTGCAAGACATGCTGTGGCAGCTGGACCTGAGCCCCGGG
TGCTAATAG
(SEQ ID NO: 23)
GATCCTATTAGCACCCGGGGCTCAGGTCCAGCTGCCACAGCATGTCTTGCAGAGACC
 
(SEQ ID NO: 24)
CCTGCAGCCTGCTCAGGGCCACCACCTCTGTAGAATAGCCTGAGGCTTCCAGGACTCCC
(SEQ ID NO: 25)
CCCAGACTGTCCAATGTCTCCAGGCCACTGGCCCATGGCAAGTGGCAACTTTTAGAGAAGG
(SEQ ID NO: 26)
CCAGCACGTGCAGCAGATCCCGCAGGTTCTCCAGGTCGTTAGATATTTGTATCACGTTT
 Oligonucleotides 16 - 22 were used to generate an approximately 220 base-pair segment which extends from the NdeI site to the XbaI site at position 220 within the coding sequence. The oligonucleotides 23 - 29 were used to generate an approximately 240 base-pair segment which extends from the XbaI site to the BamHI site.

To assemble the 220 and 240 base-pair fragments, the respective oligonucleotides were mixed in equimolar amounts, usually at concentrations of about 1-2 picomoles per microliters. Prior to assembly, all but the oligonucleotides at the 5" -ends of the segment were phosphorylated in standard kinase buffer with T4 DNA kinase using the conditions specified by the supplier of the reagents. The mixtures were heated to 95°C and allowed to cool slowly to room temperature over a period of 1-2 hours to ensure proper annealing of the oligonucleotides. The oligonucleotides were then ligated to each other and into a cloning vector, PUC19 was used, but others are operable using T4 DNA ligase. The PUC19 buffers and conditions are those recommended by the supplier of the enzyme. The vector for the 220 base-pair fragment was digested with NdeI and XbaI, whereas the vector for the 240 base-pair fragment was digested with XbaI and BamHI prior to use. The ligation mixes were used to transform E. coli DH10B cells (commercially available from Gibco/BRL) and the transformed cells were plated on tryptone-yeast (TY) plates containing 100 µg/mL of ampicillin, X-gal and IPTG. Colonies which grow up overnight were grown in liquid TY medium with 100 µg/mL of ampicillin and were used for plasmid isolation and DNA sequence analysis. Plasmids with the correct sequence were kept for the assembly of the complete gene. This was accomplished by gel-purification of the 220 base-pair and the 240 base-pair fragments and ligation of these two fragments into PUC19 linearized with NdeI and BamHI. The ligation mix was transformed into E. coli DH10B cells and plated as described previously. Plasmid DNA was isolated from the resulting transformants and digested with NdeI and BglII. The large vector fragment was gel-purified and ligated with a approximately 195 base-pair segment which was assembled as described previously from six chemically synthesized oligonucleotides as show below. 
(SEQ ID NO: 27)
TAT GCG GGT ACC GAT CCA GAA AGT TCA GGA CGA CAC CAA AAC CCT
GAT CAA AAC CAT CGT TAC
(SEQ ID NO: 28)
GCG TAT CAA CGA CAT CTC CCA CAC CCA GTC CGT GAG CTC CAA ACA
GAA GGT TAC CGG TCT GGA CTT CAT CCC GG
(SEQ ID NO: 29)
GTC TGC ACC CGA TCC TGA CCC TGT CCA AAA TGG ACC AGA CCC TGG
CTG TTT ACC AGC A
(SEQ ID NO: 30)
ATA CGC GTA ACG ATG GTT TTG ATC AGG GTT TTG GTG TCG TCC TGA
ACT TTC TGG ATC GGT ACC CGC A
(SEQ ID NO: 31)
TGC AGA CCC GGG ATG AAG TCC AGA CCG GTA ACC TTC TGT TTG GAG
CTC ACG GAC TGG GTG TGG GAG ATG TCG TTG
(SEQ ID NO: 32)
GAT CTG CTG GTA AAC AGC CAG GGT CTG GTC CAT TTT GGA CAG GGT
CAG GAT CGG G
 The ligation was transformed into E. coli cells as described previously.

The DNA from the resulting transformants was isolated and the sequence was verified by DNA sequence analysis. The plasmid with the correct sequence was digested with NdeI and BamHI and the approximately 450 base-pair insert was recloned into an expression vector.

The protein was expressed in E.coli, isolated and was folded either by dilution into PBS or by dilution into 8M urea (both containing 5 mM cysteine) and exhaustive dialysis against PBS. Following final purification of the proteins by size exclusion chromatography the proteins were concentrated to 3-3.5 mg/mL in PBS. Amino acid composition was confirmed.

### Preparation 5

The protein of SEQ ID NO: 4 with a Met Arg leader sequence was expressed in E.coli, isolated and folded as described previously. The Met Arg leader sequence was cleaved by the addition of 6-10 milliunits dDAP per mg of protein. The conversion reaction was allowed to proceed for 2-8 hours at room temperature. The progress of the reaction was monitored by high performance reversed phase chromatography. The reaction was terminated by adjusting the pH to 8 with NaOH. The des(Met-Arg) protein was further purified by cation exchange chromatography in 7-8M urea and size exclusion chromatography in PBS. Following final purification of the proteins by size exclusion chromatography the proteins were concentrated to 3-3.5 mg/mL in PBS.

Preferably, the DNA sequences are expressed with a dipeptide leader sequence encoding Met-Arg or Met-Tyr as described in U.S. Patent No. 5,126,249, herein incorporated by reference. This approach facilitates the efficient expression of proteins and enables rapid conversion to the active protein form with Cathepsin C or other dipeptidylpeptidases. The purification of proteins is by techniques known in the art and includes reverse phase chromatography, affinity chromatography, and size exclusion.

The following examples are provided merely to further illustrate the preparation of the formulations of the invention. The scope of the invention is not construed as merely consisting of the following examples.

### Example 1

The lyophilizated formulation for a protein of SEQ ID NO: 4 is for 600 mL preparation. The formulation was prepared at controlled room temperature, 20-25°C. Water for injection, 480 grams, at 20°C was added to 1000 mL beaker. The solution was stirred at 275 rpm using a Lightnin Mixer equipped with a Teflon coated three blade propeller, 4.6 cm in diameter. Dibasic sodium phosphate heptahydrate, 0.165 grams, and high purity sucrose with low endotoxin level, 27.02 grams, were added respectively, and stirred, approximately 15 minutes, until dissolved. The pH was checked and was approximately 8.3. The pH of the solution was checked and adjusted to pH 7.8 with 10% HCl. Sterile water (water for injection) was added to result in 610.8 grams of solution. The solution was sterile filtered through a 0.22 µm Millipak 20 filter using a 5 L pressure vessel at 20 psi. The solution concentration of protein was determined by UV spectroscopy, εₘₐₓ = 0.533 AU•(mg/mL)⁻¹•cm⁻¹. The fill volume was adjusted to yield 16 mg of protein per vial. The vials were fitted with stoppers for lyophilization and loaded at a shelf temperature of 5°C. After loading the shelf temperature was adjusted to -5°C and held for 1 hour. Complete freezing was achieved by adjusting the shelf temperature to -45°C and waiting until the thermocouples read -35°C or less for 1 hour. Lyophilization proceeds at a shelf temperature of -20°C and pressure of 85 microns. Primary drying was complete when the thermocouples reached -22°C and maintained for 4 hours. Secondary drying was initiated by setting the shelf temperature to 0°C hold 1 hour, then adjusting the shelf temperature to +35°C. The shelf temperature was held at 35°C until the thermocouples reached and maintained 30°C for 4 hours. After secondary drying, the chamber was back filled with nitrogen gas and the vials stoppered and capped.

### Example 2

To generate a soluble formulation containing a protein of Seq ID NO: 4 (hereafter, Protein NO: 4) the lyophilized solid bulk material was first dissolved in water containing a buffer, e.g., arginine (11 mM), at alkaline pH conditions (stock 1). The concentration of Protein NO: 4 (22 mg/mL) is verified by UV/VIS spectrophotometry using the known extinction coefficient for Protein NO: 4 at the maximum spectral absorbance (279 nm or 280 nm), measuring the maximum spectral absorbance (279 nm or 280 nm), and utilizing the dilution factor. Stock solutions containing sucrose, and the preservative, m-cresol, were prepared by dissolving the solid in water (stocks 2 and 3, respectively). A formulation containing Protein NO: 4 at 10 mg/mL was prepared by addition of an aliquot of stock 1 (4.5 mL) to a 10 mL volumetric flask. Aliquots of stocks 2 (1.0 mL), stock 3 (3.0 mL), and water were added in order. Enough water was added to adjust the final volume to 10 mL. The pH of the solution was checked and, if necessary, the pH was adjusted with µL quantities of dilute HCl or dilute NaOH to a pH 8.5 (± 0.1). The final solution condition was 10 mg/mL of Protein NO: 4, 0.3 % m-cresol, and 9 or 15 % sucrose at pH 8.5 (± 0.1). The solution is then distributed into vials and stored at the desired temperatures.

The principles, preferred embodiments and modes of operation of the present invention have been described in the foregoing specification. The invention which is intended to be protected herein, however, is not to be construed as limited to the particular forms disclosed, since they are to be regarded as illustrative rather than restrictive. Variations and changes may be made by those skilled in the art without departing from the spirit of the invention.

## Claims

1. A lyophilized composition, comprising an obesity protein, and a sugar selected from the group consisting of sucrose, trehalose, or a mixture thereof.

2. A composition of Claim 1 wherein the protein is a protein of SEQ ID NO: 2:

(SEQ ID NO: 2)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Trp Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys
said protein optionally having a leader sequence.

3. A composition of Claim 1 wherein the protein is a protein of the Formula (I): 
(SEQ ID NO: 1)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Xaa Ser Val Ser Ser

35 40 45
Lys Gln Lys Val Thr Gly Leu Asp Phe Ile Pro Gly Leu His Pro Ile
 
50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Trp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys                                                     (I)
 wherein:
Xaa at position 28 is Gln or absent;
said protein having at least one of the following substitutions:
Gln at position 4 is replaced with Glu;
Gln at position 7 is replaced with Glu;
Asn at position 22 is replaced with Gln or Asp;
Thr at position 27 is replaced with Ala;
Xaa at position 28 is replaced with Glu;
Gln at position 34 is replaced with Glu;
Met at position 54 is replaced with methionine sulfoxide, Leu, Ile, Val, Ala, or Gly;
Gln at position 56 is replaced with Glu;
Gln at position 62 is replaced with Glu;
Gln at position 63 is replaced with Glu;
Met at position 68 is replaced with methionine sulfoxide, Leu, Ile, Val, Ala, or Gly;
Asn at position 72 is replaced with Gln, Glu, or Asp; Gln at position 75 is replaced with Glu;
Ser at position 77 is replaced with Ala;
Asn at position 78 is replaced with Gln or Asp;
Asn at position 82 is replaced with Gln or Asp;
His at position 97 is replaced with Gln, Asn, Ala, Gly, Ser, or Pro;
Trp at position 100 is replaced with Ala, Glu, Asp, Asn, Met, Ile, Phe, Tyr, Ser, Thr, Gly, Gln, Val or Leu;
Ala at position 101 is replaced with Ser, Asn, Gly, His, Pro, Thr, or Val;
Ser at position 102 is replaced with Arg;
Gly at position 103 is replaced with Ala;
Glu at position 105 is replaced with Gln;
Thr at position 106 is replaced with Lys or Ser;
Leu at position 107 is replaced with Pro;
Asp at position 108 is replaced with Glu;
Gly at position 111 is replaced with Asp;
Gly at position 118 is replaced with Leu;
Gln at position 130 is replaced with Glu;
Gln at position 134 is replaced with Glu;
Met at position 136 is replaced with methionine sulfoxide, Leu, Ile, Val, Ala, or Gly;
Trp at position 138 is replaced with Ala, Glu, Asp, Asn, Met, Ile, Phe, Tyr, Ser, Thr, Gly, Gln, Val or Leu; or
Gln at position 139 is replaced with Glu; or a pharmaceutically acceptable salt thereof.

4. A pharmaceutical formulation comprising an obesity protein and a sugar selected from the group consisting of sucrose, trehalose, or a mixture thereof.

5. A formulation of Claim 4, wherein said protein is the protein is a protein of SEQ ID NO: 2: 
(SEQ ID NO: 2)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu
 
                    85                                      90                                      95
Glu Asn Leu Arg Asp Leu Leu His Val Leu Ala Phe Ser Lys Ser Cys

                100                                     105                                     110
His Leu Pro Trp Ala Ser Gly Leu Glu Thr Leu Asp Ser Leu Gly Gly

            115                                     120                                     125
Val Leu Glu Ala Ser Gly Tyr Ser Thr Glu Val Val Ala Leu Ser Arg

    130                                     135                                     140
Leu Gln Gly Ser Leu Gln Asp Met Leu Trp Gln Leu Asp Leu Ser Pro

145
Gly Cys
 said protein optionally having a leader sequence.

6. A formulation of Claim 4, wherein the protein is a protein of the Formula (I): 
(SEQ ID NO: 1)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Xaa Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Trp Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys (I)
 wherein:
Xaa at position 28 is Gln or absent;
said protein having at least one of the following substitutions:
Gln at position 4 is replaced with Glu;
Gln at position 7 is replaced with Glu;
Asn at position 22 is replaced with Gln or Asp;
Thr at position 27 is replaced with Ala;
Xaa at position 28 is replaced with Glu;
Gln at position 34 is replaced with Glu;
Met at position 54 is replaced with methionine sulfoxide, Leu, Ile, Val, Ala, or Gly;
Gln at position 56 is replaced with Glu;
Gln at position 62 is replaced with Glu;
Gln at position 63 is replaced with Glu;
Met at position 68 is replaced with methionine sulfoxide, Leu, Ile, Val, Ala, or Gly;
Asn at position 72 is replaced with Gln, Glu, or Asp;
Gln at position 75 is replaced with Glu;
Ser at position 77 is replaced with Ala;
Asn at position 78 is replaced with Gln or Asp;
Asn at position 82 is replaced with Gln or Asp;
His at position 97 is replaced with Gln, Asn, Ala, Gly, Ser, or Pro;
Trp at position 100 is replaced with Ala, Glu, Asp, Asn, Met, Ile, Phe, Tyr, Ser, Thr, Gly, Gln, Val or Leu;
Ala at position 101 is replaced with Ser, Asn, Gly, His, Pro, Thr, or Val;
Ser at position 102 is replaced with Arg;
Gly at position 103 is replaced with Ala;
Glu at position 105 is replaced with Gln;
Thr at position 106 is replaced with Lys or Ser;
Leu at position 107 is replaced with Pro;
Asp at position 108 is replaced with Glu;
Gly at position 111 is replaced with Asp;
Gly at position 118 is replaced with Leu;
Gln at position 130 is replaced with Glu;
Gln at position 134 is replaced with Glu;
Met at position 136 is replaced with methionine sulfoxide, Leu, Ile, Val, Ala, or Gly;
Trp at position 138 is replaced with Ala, Glu, Asp, Asn, Met, Ile, Phe, Tyr, Ser, Thr, Gly, Gln, Val or Leu; or
Gln at position 139 is replaced with Glu;
or a pharmaceutically acceptable salt thereof.

7. A pharmaceutical formulation, comprising the lyophilized composition of any one of Claims 1 through 3 and water.

8. A therapeutically useful kit, comprising a sterile vial containing a lyophilized composition of any one of Claims 1 through 3, and a pharmaceutically acceptable diluent for reconstitution thereof.

9. A kit of Claim 7, which further comprises a device suitable for injection.

10. A kit of Claim 8 or 9, wherein the diluent comprises sterile water, a physiologically tolerated buffer, and optionally a pharmaceutically acceptable preservative.

11. A formulation of any one of Claims 4 through 6 for use in the treatment of obesity.

12. A process of preparing a composition of any one of Claims 1 through 3, which comprises combining water, an obesity protein analog, and a sugar selected from the group consisting of sucrose, trehalose, or a mixture thereof; and lyophilizing the resulting solution.


SEQUENCE LISTING

(1) GENERAL INFORMATION:

(i) APPLICANT: ELI LILLY AND COMPANY
(B) STREET: Lilly Corporate Center
(C) CITY: Indianapolis
(D) STATE: United States of America
(F) ZIP: 46285

(ii) TITLE OF INVENTION: FORMULATIONS OF OB PROTEIN

(iii) NUMBER OF SEQUENCES: 32

(iv) CORRESPONDENCE ADDRESS:
(A) ADDRESSEE: C. M. Hudson
(B) STREET: Erl Wood Manor
(C) CITY: Windlesham
(D) STATE: Surrey
(E) COUNTRY: United Kingdom
(F) ZIP: GU20 6PH

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:
(A) APPLICATION NUMBER: 97301995.3
(B) FILING DATE: 24th March 1997

(2) INFORMATION FOR SEQ ID NO:1:

(i) SEQUENCE CHARACTERISTICS:
(A) LENGTH: 146 amino acids
(B) TYPE: amino acid
(C) STRANDEDNESS: double
(D) TOPOLOGY: linear

(ii) MOLECULE TYPE: protein

(ix) FEATURE:
(A) NAME/KEY: Protein
(B) LOCATION: 1
(D) OTHER INFORMATION: /note= "XAA AT POSITION 28 IS GLN
OR ABSENT"

(xi) SEQUENCE DESCRIPTION: SEQ ID NO:1:

Val Pro Ile Gln Lys Val Gln Asp Asp Thr Lys Thr Leu Ile Lys Thr
                5                   10                  15

Ile Val Thr Arg Ile Asn Asp Ile Ser His Thr Xaa Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:2:

     (i) SEQUENCE CHARACTERISTICS:
          (A) LENGTH: 146 amino acids
          (B) TYPE: amino acid
          (C) STRANDEDNESS: double
          (D) TOPOLOGY: linear

    (ii) MOLECULE TYPE: protein

    (xi) SEQUENCE DESCRIPTION: SEQ ID NO:2:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:3:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO:3:

Val Pro Ile Gln Lys Val Gln Asp Asp Thr Lys Thr Leu Ile Lys Thr
1               5                   10                  15

Ile Val Thr Arg Ile Asn Asp Ile Ser His Ala Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:4:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:4:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Ala Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:5:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:5:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Gln Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:6:

(i) SEQUENCE CHARACTERISTICS:
(A) LENGTH: 146 amino acids
(B) TYPE: amino acid

(C) STRANDEDNESS: single
(D) TOPOLOGY: linear

(ii) MOLECULE TYPE: protein

(xi) SEQUENCE DESCRIPTION: SEQ ID NO:6:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO:7:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:7:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Gln Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO:8:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO:8:

Val Pro Ile Gln Lys Val Gln Asp Asp Thr Lys Thr Leu Ile Lys Thr
1               5                   10                  15

Ile Val Thr Arg Ile Asn Asp Ile Ser His Ala Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Ala Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:9:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO:9:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Ala Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO:10:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO:10:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

Ser Leu Pro Gln Thr Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO:11:

(i) SEQUENCE CHARACTERISTICS:
(A) LENGTH: 84 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:11:

GTGCTGGCCT TCTCTAAAAG TTGCCACTTG CCAGCTGCCA GTGGCCTGGA GACATTGGAC 60

AGTCTGGGGG GAGTCCTGGA AGCC 84

(2) INFORMATION FOR SEQ ID NO:12:

(i) SEQUENCE CHARACTERISTICS:
(A) LENGTH: 87 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:12:

TGAGGCTTCC AGGACTCCCC CCAGACTGTC CAATGTCTCC AGGCCACTGG CAGCTGGCAA 60

GTGGCAACTT TTAGAGAAGG CCAGCAC 87

(2) INFORMATION FOR SEQ ID NO:13:

(i) SEQUENCE CHARACTERISTICS:
(A) LENGTH: 66 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:13:

TATGAGGGTA CCTATCCAAA AAGTACAAGA TGACACCAAA ACACTGATAA AGACAATAGT 60

CACAAG 66

(2) INFORMATION FOR SEQ ID NO:14:

(i) SEQUENCE CHARACTERISTICS:
(A) LENGTH: 72 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:14:

GATAGATGAT ATCTCACACA CACAGTCAGT CTCATCTAAA CAGAAAGTCA CAGGCTTGGA 60

CTTCATACCT GG 72

(2) INFORMATION FOR SEQ ID NO:15:

(i) SEQUENCE CHARACTERISTICS:
(A) LENGTH: 77 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:15:

GCTGCACCCC ATACTGACAT TGTCTAAAAT GGACCAGACA CTGGCAGTCT ATCAACAGAT 60

CTTAACAAGT ATGCCTT 77

(2) INFORMATION FOR SEQ ID NO:16:

(i) SEQUENCE CHARACTERISTICS:
(A) LENGTH: 38 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:16:

CTAGAAGGCA TACTTGTTAA GATCTGTTGA TAGACTGC 38

(2) INFORMATION FOR SEQ ID NO:17:

(i) SEQUENCE CHARACTERISTICS:
(A) LENGTH: 62 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:17:

CAGTGTCTGG TCCATTTTAG ACAATGTCAG TATGGGGTGC AGCCCAGGTA TGAAGTCCAA 60

GC 62

(2) INFORMATION FOR SEQ ID NO:18:

(i) SEQUENCE CHARACTERISTICS:
(A) LENGTH: 83 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:18:

CTGTGACTTT CTGTTTAGAT GAGACTGACT GTGTGTGTGA GATATCATCT ATCCTTGTGA 60

CTATTGTCTT TATCAGTGTT TTG 83

(2) INFORMATION FOR SEQ ID NO:19:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:19:

GTGTCATCTT GTACTTTTTG GATAGGTACC CTCA 34

(2) INFORMATION FOR SEQ ID NO:20:

(i) SEQUENCE CHARACTERISTICS:
(A) LENGTH: 92 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:20:

CTAGAAACGT GATACAAATA TCTAACGACC TGGAGAACCT GCGGGATCTG CTGCACGTGC 60

TGGCCTTCTC TAAAAGTTGC CACTTGCCAT GG 92

(2) INFORMATION FOR SEQ ID NO:21:

(i) SEQUENCE CHARACTERISTICS:
(A) LENGTH: 74 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:21:

GCCAGTGGCC TGGAGACATT GGACAGTCTG GGGGGAGTCC TGGAAGCCTC AGGCTATTCT 60

ACAGAGGTGG TGGC 74

(2) INFORMATION FOR SEQ ID NO:22:

(i) SEQUENCE CHARACTERISTICS:
(A) LENGTH: 70 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:22:

CCTGAGCAGG CTGCAGGGGT CTCTGCAAGA CATGCTGTGG CAGCTGGACC TGAGCCCCGG 60

GTGCTAATAG 70

(2) INFORMATION FOR SEQ ID NO:23:

(i) SEQUENCE CHARACTERISTICS:
(A) LENGTH: 57 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:23:

GATCCTATTA GCACCCGGGG CTCAGGTCCA GCTGCCACAG CATGTCTTGC AGAGACC 57

(2) INFORMATION FOR SEQ ID NO:24:

(i) SEQUENCE CHARACTERISTICS:
(A) LENGTH: 59 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:24:

CCTGCAGCCT GCTCAGGGCC ACCACCTCTG TAGAATAGCC TGAGGCTTCC AGGACTCCC 59

(2) INFORMATION FOR SEQ ID NO:25:

(i) SEQUENCE CHARACTERISTICS:
(A) LENGTH: 61 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:25:

CCCAGACTGT CCAATGTCTC CAGGCCACTG GCCCATGGCA AGTGGCAACT TTTAGAGAAG 60

G 61

(2) INFORMATION FOR SEQ ID NO:26:

(i) SEQUENCE CHARACTERISTICS:
(A) LENGTH: 59 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:26:

CCAGCACGTG CAGCAGATCC CGCAGGTTCT CCAGGTCGTT AGATATTTGT ATCACGTTT 59

(2) INFORMATION FOR SEQ ID NO:27:

(i) SEQUENCE CHARACTERISTICS:
(A) LENGTH: 63 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:27:

TATGCGGGTA CCGATCCAGA AAGTTCAGGA CGACACCAAA ACCCTGATCA AAACCATCGT 60

TAC 63

(2) INFORMATION FOR SEQ ID NO:28:

(i) SEQUENCE CHARACTERISTICS:
(A) LENGTH: 74 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:28:

GCGTATCAAC GACATCTCCC ACACCCAGTC CGTGAGCTCC AAACAGAAGG TTACCGGTCT 60

GGACTTCATC CCGG 74

(2) INFORMATION FOR SEQ ID NO:29:

(i) SEQUENCE CHARACTERISTICS:
(A) LENGTH: 58 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:29:

GTCTGCACCC GATCCTGACC CTGTCCAAAA TGGACCAGAC CCTGGCTGTT TACCAGCA 58

(2) INFORMATION FOR SEQ ID NO:30:

(i) SEQUENCE CHARACTERISTICS:
(A) LENGTH: 67 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:30:

ATACGCGTAA CGATGGTTTT GATCAGGGTT TTGGTGTCGT CCTGAACTTT CTGGATCGGT 60

ACCCGCA 67

(2) INFORMATION FOR SEQ ID NO:31:

(i) SEQUENCE CHARACTERISTICS:
(A) LENGTH: 75 base pairs
(B) TYPE: nucleic acid
(C) STRANDEDNESS: single

(D) TOPOLOGY: linear

(ii) MOLECULE TYPE: cDNA

(xi) SEQUENCE DESCRIPTION: SEQ ID NO:31:

TGCAGACCCG GGATGAAGTC CAGACCGGTA ACCTTCTGTT TGGAGCTCAC GGACTGGGTG 60

TGGGAGATGT CGTTG 75

(2) INFORMATION FOR SEQ ID NO:32:

(i) SEQUENCE CHARACTERISTICS:
(A) LENGTH: 55 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:32:

GATCTGCTGG TAAACAGCCA GGGTCTGGTC CATTTTGGAC AGGGTCAGGA TCGGG 55